# EUROPEAN PATENT APPLICATION

(11) **EP 3 701 980 A1**
(43) Date of publication of application: **02.09.2020**
(21) Application number: 18870644.4
(22) Date of filing: 29.03.2018
(51) Int. Cl.: A61M 5/142, A61B 5/00

(54) **SYSTEM FOR REMOTELY MONITORING AND CONTROLLING THE ADMINISTRATION OF MEDICINAL PRODUCTS**

(30) Priority: 24.10.2017 BR 102017022893
(71) Applicant: Ambrósio, Cláudio, Afonso, 36880-000 Muriaé, Minas Gerais (BR)
(72) Inventor: Ambrósio, Cláudio, Afonso, 36880-000 Muriaé, Minas Gerais (BR)
(74) Representative: ZBM Patents - Zea, Barlocci & Markvardsen
(86) International application number: PCT/BR2018/050086
(87) International publication number: WO 2019/079868

(57) **Abstract**

This invention provides a system for remotely monitoring and controlling medication delivery, and the aforementioned system comprises an infusion pump (1), a device for monitoring and measuring physiological characteristics of an individual (2) configured to continuously monitor at least one physiological characteristic of the individual; a device for remote monitoring and control (3) to remotely monitor and control the infusion pump and the device for monitoring and measuring the physiological characteristics of an individual, through a communication network (5); a communication device (4) configured to communicate with the remote monitoring and control device, through one communication network; and in which said device for monitoring and measuring an individual's physiological characteristics (2) sends information form such physiological characteristics of an individual to the remote monitoring and control device (3) through a communication network (5).

## Description

### FIELD OF INVENTION

This invention is related to a system that is able to provide not only a monitoring but also a medication remote delivery control.

### GROUNDS OF THE INVENTION

The use of increasingly advanced and complex technologies in the medical field has been a growing trend. Thinking about the treatment of a disease is not enough, without that involving comfort and the preservation of the individual's life quality.

The need for treatment requiring hospitalization causes enormous changes and perhaps disturbances in the habits of a patient's life, moving him/her away from social interaction with his/her loved ones and friends, his/her personal belongings, and increasing the risk of hospital infection.

It should also be noted that staying at home and socializing with family members minimizes the discomfort caused by an illness and its treatment, because long or frequent periods of hospitalization, in addition to being extremely unpleasant and stressful for all the individuals involved and the patient, can represent a threat to such individuals.

As regards a patient suffering from cancer, for example, the presence of cancer cells has a great impact on his/her daily life, leading to profound changes in his/her usual way of life, in addition to bringing about side effects, often harmful, in terms of compromising the capacity and ability to carry out daily activities.

In certain cases, such as a patient having hypoglycemia, which does not require continuous hospitalization, the disease symptoms may not be pre-identified by an individual, who may suffer mental confusion or loss of consciousness making him/her incapable of self-medication.

Consequently, there is a concern in ensuring both proper treatment and patient life quality. There is a need for a system that is able to monitor a patient's physiological condition and continuously monitor the appropriate treatment on the basis of information on his/her physiological condition, either automatically or through the remote interaction of an individual. There is a need for a system that is able to provide proper treatment without affecting the patient's life quality.

### INVENTION PURPOSE

This invention is intended to provide a system that is able to provide not only monitoring, but also remote control of medication delivery to patients.

### INVENTION SUMMARY

This invention provides a system for remote monitoring and control of medication delivery, comprising:
an infusion pump;
a device for monitoring and measuring physiological features of an individual configured to continuously monitor at least one physiological feature of the individual;
a remote monitoring and control device for remotely monitoring and controlling the infusion pump and the monitoring device and measuring the physiological features of an individual, through a communication network;
a communication device setup to communicate with the remote monitoring and control device via a communication network; and
in which said monitoring and measuring device of physiological characteristics of an individual sends information of those physiological features of an individual to the monitoring and remote control device through a communication network.

In a preferred embodiment of the system according to this invention, the system comprises communication means between the monitoring and measuring device of the physiological characteristics of an individual and the remote monitoring and control device, since the monitoring and remote control device receives information from the monitoring and measuring device of an individual's physiological characteristics; means for communication between the infusion pump and the remote monitoring and control device, where such a remote monitoring and control device triggers the infusion pump and sends command information for medication delivery; communication means between the communication device and the remote monitoring and control device, where the remote monitoring and control device is configured to receive and transmit information to/from the communication device.

In a preferred embodiment of the system according to this invention, the infusion pump is a portable infusion pump and comprises at least one catheter and at least one medication compartment.

In a preferred embodiment of the system according to the present invention, the device for monitoring and measuring the physiological features of an individual comprises at least one cell phone chip, at least one communication interface, a GPS receiver module and a control panel consisting of a digital display and command keys.

In a preferred embodiment of the system according to this invention, the remote monitoring and control device is configured to allow the user to interact remotely, via the communication device, sending and receiving information to/from that remote monitoring and control device, through a communication network.

In a preferred embodiment of the system according to this invention, the information sent and/or received by the user from the communication device, via the remote monitoring and control device, is at least one infusion pump trigger information, medication administration information, audio and/or visual information, text information, phone call, sending and receiving SMS messages, infusion pump battery level information, battery level information of the device monitoring and measuring an individual's physiological characteristics, information on the patient's location.

In a preferred embodiment of the system according to this invention, the remote monitoring and control device automatically monitors and controls the infusion pump based on the result obtained by the said monitoring device and measurement of physiological characteristics of an individual.

In a preferred embodiment of the system according to this invention, said device of remote monitoring and controlling sends the infusion pump activation information, medication administration information, audio and/or visual information, text information, phone call, SMS message, patient location information, based on the result obtained by the device for monitoring and measuring physiological characteristics of an individual.

### DRAWING OVERVIEW

The goals, technical effects and advantages of this invention will be apparent to those aware of the state of the art from the following detailed description, making reference to the enclosed figure showing the preferred form of embodiment and exemplifying, but not limited to the invention.

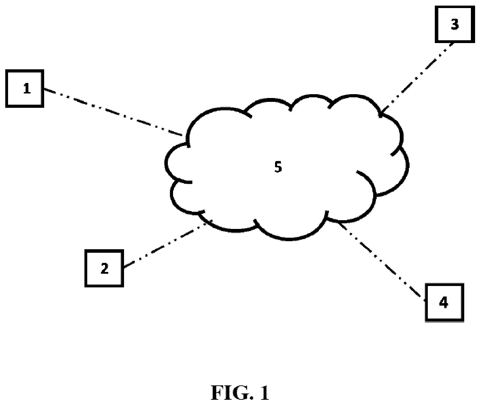

Figure 1 illustrates a system for remote monitoring and controlling of medication delivery according to a particular form of performing this invention.

### DETAILED INVENTION DESCRIPTION

According to a form of embodiment, the system to provide remote monitoring and control of medication delivery includes at least one infusion pump, at least one device for monitoring and measuring the individual's physiological characteristics, at least one device for remote monitoring and controlling and at least one communication device, all connected via a communication network.

Any of the above-mentioned devicescan operate in the stand-alone mode or together as part of the same module. Likewise, any of the devices can be purchased separately in order to complement other systems with other applications.

A communication network means any network capable of transmitting information (voice, data and/or signals), whether it is a wired communication network or a wireless communication network. In an example applied to this invention, the communication network can be a wireless network, like a cellular telephone communication network.

Such an infusion pump can be a commonly used infusion pump. In particular, the infusion pump of this invention can be a small-sized portable infusion pump. The small size of the portable infusion pump is intended to provide convenience and comfort to the patient. Additionally, the portable infusion pump of this invention includes at least one catheter and at least one medication compartment. The medication compartment can be a compartment for any type of medication, like antibiotics, painkillers, chemotherapeutic agents, among others.

The monitoring and measuring device of an individual's physiological features may be any of the known devices available in the market. For example, such a device could be a blood glucose monitor, which is configured to be used in a patient's body. In general, such monitors are placed in a specific area of a patient's body, with a sensor inserted under the skin, in such a way that the sensor can outreach the patient's body fluids and measure the physiological characteristics. Such monitors can be configured to make measurement on a continuous or on a periodic basis such as every hour, half an hour, every ten minutes, every five minutes, or such other desired time increment.

Such a device for monitoring and measuring the physiological characteristics of an individual of this invention can also be connected in a communicative way with one or more members, comprising a patient's care network, via a remote monitoring and control device. Therefore, the so-called monitoring and measuring devices of physiological characteristics of an individual and remote monitoring and control device present characteristics of a communication device, including, for example, at least one mobile phone chip, at least one interface for communication, e.g. Internet communication, Bluetooth, Wi-Fi, among others. Such a device may additionally have a GPS (*Global Positioning System)* receiver module so that members of the patient care network can use the patient's precise location information. In addition, the remote monitoring and control device can be provided with a control panel consisting of a digital display and command keys to enable access and visualization of the information by the patient himself, provided by such a device for monitoring and measuring physiological characteristics.

Furthermore, the remote monitoring and control device and the infusion pump may be comprised within the same module, each with its own respective function.

In another form of embodiment, the members of the patient's care network may be one or more of several emergency contacts of the patient such as family members, friends, co-workers and other personal acquaintances of the patient. The members of the patient's care network may also be primary care doctors, emergency medical staff or even nursing attendants, other caregivers or even assigned or colleagues. In addition, the patient him/herself may have his/her communication device belonging to such a patient care network.

In a generic sense, each member of the patient's care network may well be also a user employing a communication device communicating with the remote monitoring and control device in order to receive/send information on the patient's physiological features , send command information, text audio and/or visual information, alarm(s), telephone call, SMS (*Short Message Service*) message, infusion pump battery level information and measuring of physiological characteristics, patient's location information, as well as accessing data stored by the remote monitoring and control device.

The abovementioned communication device may be one among cellular telephone, tablet, laptop, PC or any other electronic device that can receive and transmit data from the remote monitoring and control device.

Those members of the patient's care network such as doctors, nurses and other medical professionals and caretakers, may also access all data on the patient's physiological features to treatments already given. Thus, such members can plan the patient's treatment more efficiently and without surprises.

Furthermore, in another form of accomplishment, the patient's physiological features, as well as the treatments already delivered and of all data from any system module may be stored in the remote monitoring and control device, in its own storage hardware or in a external server, accessible by any communication network for future reference, such as for example, a *cloud* server. In particular, this may allow such members of the patient care network to treat efficiently and effectively a patient, taking advantage of the information about the patient's performance and response in view of the applied treatment.

As an example, the stored information may be information on the number of doses already given, and such information may be related to the schedule and patient's conditions at a given time.

In another embodiment, such remote monitoring and control device may be configured to emit alerts to the patient him/herself or to the care network members, about changes in the patient's physiological features through audio and/or visual information, text information, information in the form of alert/alarm, telephone call or even SMS message. Thus, for example, the monitor can report an alarm to one or more emergency contacts so that these are informed of the patient's condition and can take proper action, whether they are sent remotely, at a distance, through the remote monitoring and control device or even going to the location where the patient is.

Still in another embodiment, the remote monitoring and control device can send the patient's geographic location to his/her contact network, which will be able to visualize it through an interaction interface of their mobile devices. In addition, each contact in that network can open a communication channel with the patient in case of emergencies and from that, listen to everything that happens in the environment where the patient is and communicate with him/her. This can be useful for people with hypoglycemia, epilepsy, childhood asthma attacks, migraine attacks, heart attacks such as angina attacks, elderly people with Alzheimer's attacks and other debilitating situations.

In another embodiment of the invention, the treatment may occur automatically. For example, the device for monitoring and measuring physiological characteristics of an individual may be connected, in a communicative way, to a portable infusion pump via the remote monitoring and control device, all the equipment is communicatively connected through a communication network.

As an example that can be applied to this invention, continuous or periodic insulin treatments are often prescribed for the purpose of suppressing excess glucose from the diabetic patient's body, however, when a patient experiences hypoglycemia, the patient's blood glucose levels are too low and leave them at risk of dangerous symptoms, such as dizziness, blurred vision, confusion, and even coma. Thus, in another embodiment of the invention, when such a monitoring and measuring device of physiological characteristics of an individual measures glucose levels in the patient's blood below a threshold, it communicates with the remote monitoring and control device so that it automatically sends instructions to begin the patient's treatment, activating the infusion pump whenever necessary.

The invention provides communicatively such a device for monitoring and measuring physiological characteristics of an individual with doctors, hospitals and/or emergency contacts defining a patient's care network. Thus, it is able to alert a patient and/or one or more pre-selected members of that patient's care network of those physiological characteristics of the patient.

As illustrated in Figure 1 of this invention, the system for providing remote monitoring and control of medication delivery is capable of responding to a large number of illnesses and comprises an infusion pump 1, a device for monitoring and measuring an individual's physiological characteristics **2,** a remote monitoring and control device **3,** and at least one communication device **4.** In one form of embodiment of this invention, pump **1** and some (or all) devices **2, 3** and **4** are communicatively connected though a communication network **5.**

Users of any communication devices **4** may use their devices as communicatively connected members of a patient's care network allowing them to receive physiological characteristics information of the monitored individual and measured by device **2,** and, if necessary, issue a response in the event of an emergency. In the form of the exemplary embodiment of Figure 1, such a patient's care network can be improved and facilitated by the remote monitoring and control device **3.** As such, device **3** facilitates and improves interaction between a group of members, as pre-established by the patient him/herself, such as physicians, emergency contacts, and other of his/her caregivers. For the purpose of the exemplary accomplishment of Figure 1, the group of members comprising that patient's care network includes the users of communication devices **4,** for example, the mobile device users.

Additionally, the said remote monitoring and control device **3** can implement the system to provide remote monitoring and control of medication delivery, by doing it automatically. Members of the patient's care network can access such a device **3** using their communication devices **4** and may obtain information regarding the individual physiological characteristics, as well as procedures already carried out through said system.

Although embodiments and examples have been described above, it should be noted that they are used only to illustrate this invention, but they have no intention to limit it. Several modifications, improvements, and equivalent can be made by those who know well the state of the art without escaping the scope of this invention. Such modifications, improvements, and equivalent should also be considered within the scope of protection of this invention.

## Claims

1. Remote monitoring and control system for medication delivery, **characterized by** comprising:
an infusion pump (1);
a device for monitoring and measuring an individual's physiological characteristics (2) configured to continuously monitor at least one physiological characteristic of the individual;
a remote monitoring and control device (3) to remotely monitor and control the infusion pump (1) and the measurement of physiological characteristics of an individual (2), through a communication network (5); and
a communication device (4) configured to communicate with the remote monitoring and control device (3) through a communication network (5),
in which the said monitoring and measuring device of physiological characteristics of an individual (2) sends information of those physiological characteristics of an individual to the monitoring device and remote control (3) through a communication network (5).

2. A system, according to claim 1, which is **characterized by** comprising:
communication means between the device for monitoring and measuring physiological characteristics of an individual (2) and the device for remote monitoring and control (3) where the remote monitoring and control device (3) receives information from the monitoring and measuring device of physiological characteristics of an individual (2);
communication means between the said infusion pump (1) and the remote monitoring and control device (3), in which the remote monitoring and control device (3) triggers the said infusion pump (1) and sends command information for medication delivery;
communication means between the communication device (4) and the remote monitoring and control device (3), in which the monitoring device and distance control (3) is configured to receive and transmit information to/from the communication device (4).

3. A system, according to claim 1 or 2, that is **characterized by** the infusion pump (1) being a portable infusion pump, and comprising at least one catheter and at least one medication compartment.

4. A system, according to claim 1 or 2, that is **characterized by** the device for monitoring and measuring physiological characteristics of an individual (2) comprising at least one mobile phone chip, at least one communication interface, one GPS receiver module and one control panel consisting of a digital display and command keys.

5. A system, according to claim 1 or 2, that is **characterized by** the remote monitoring and control device (3) being configured to allow a user to interact remotely, through the communication device (4), sending and receiving information to/from the remote monitoring and control device (3), through a communication network (5).

6. A system, according to claim 5, that is **characterized by** the information sent and/or received by the user from the communication device (4), through the remote monitoring and control device (3), being at least one of infusion pump trigger information (1), medication administration information, audio and/or visual information, text information, phone call, sending and receiving SMS messages, infusion pump battery level information (1), battery level information of the device monitoring and measuring physiological characteristics of an individual (2), information on the patient's location.

7. A system, according to any of the claims 1, 2, 5 or 6, that is **characterized by** the remote monitoring and control device (3) automatically monitoring and controlling the infusion pump (1) based on the result obtained by the device for monitoring and measuring physiological characteristics of an individual (2).

8. A system, according to claim 6 or 7, that is **characterized by** the remote monitoring and control device (3) sending the infusion pump actuation information (1), the information on the medication administration, audio and/or visual information, text information, telephone call, SMS message, information on the patient's location, based on the result obtained by the aforementioned device for monitoring and measuring physiological characteristics of an individual (2).
